# EUROPEAN PATENT SPECIFICATION

(11) **EP 1 404 296 B1**
(45) Date of publication and mention of the grant of the patent: **17.12.2014**
(21) Application number: 02726348.2
(22) Date of filing: 28.05.2002
(51) Int. Cl.: A61K 9/00

(54) **EFFERVESCENT FORMULATIONS OF NON-STEROIDAL ANTI-INFLAMMATORY DRUGS**
BRAUSEZUSAMMENSETZUNGEN VON NICHTSTEROIDALEN ENTZÜNDUNGSHEMMENDEN WIRKSTOFFEN
AGENTS THERAPEUTIQUES

(30) Priority: 07.06.2001 GB 0113842
(43) Date of publication of application: 07.04.2004
(73) Proprietor: Reckitt Benckiser Healthcare (UK) Limited, Slough, Berkshire SL1 3UH (GB)
(72) Inventor: HEWITT, Philip, Thomas, Nottingham NG2 3AA (GB); SHERRY, Robert, Arthur, Nottingham NG2 3AA (GB); BOOT, Jacqueline, Marie, Nottingham NG2 3AA (GB)
(74) Representative: O'Brien, Niall James
(86) International application number: PCT/GB2002/002491
(87) International publication number: WO 2002/098388

(56) References cited:
- EP-A- 0 369 228
- US-B1- 6 171 617

## Description

This invention relates to formulations containing a non-steroidal anti-inflammatory drug, to processes to prepare them and to uses thereof.

Non-steroidal anti-inflammatory drugs (NSAIDs) are a widely used class of medicaments. They are a well defined group of compounds and include phenylpropionic acids such as ibuprofen, naproxen, ketoprofen and flurbiprofen. They are primarily used for the treatment of one or more of pain, inflammation and fever, for example rheumatoid arthritis, ankylosing spondylitis, osteoarthritis, postoperative pain, post-partum pain and soft tissue injuries. One example is ibuprofen, which is available under prescription in the UK (e.g. Brufen (RTM)), generally at doses up to 3200 mg per day. Ibuprofen is also available as a non-prescription drug in the UK (e.g. Nurofen (RTM)) primarily for the treatment of symptoms of pain and fever including headache, migraine, rheumatic pain, muscular pain, backache, neuralgia, dysmenorrhoea, dental pain and colds and flu, generally at doses up to 1200 mg per day.

It is desired to formulate these NSAID medicaments in an effervescent dosage form, such as granules or tablets. These dosage forms are convenient to administer to a patient. Such formulations include an effervescent couple which reacts in the presence of water to liberate carbon dioxide. Accordingly, when the dosage form is combined with water or other suitable liquid, the effervescent reaction is initiated and the formulation is rapidly dispersed within the liquid. The patient may then drink the liquid dispersion/suspension.

In order to achieve a satisfactory effervescent formulation a number of formulation issues must be overcome. Ibuprofen and other NSAIDs are generally acidic and substantially insoluble drugs. A particular problem that has been identified is how to achieve a stable product where the effervescent couple does not react prematurely and where the acidic NSAID does not react with the basic component of the effervescent couple without requiring the presence of a significant proportion of other excipients to minimise this potential reaction in the formulation, leading to cost and manufacturing disadvantages. The NSAIDs may also be administered in relatively high doses, e.g. up to 800 mg ibuprofen or naproxen per unit dose. Thus, there is a problem to provide a dosage form which may include a large proportion of NSAID, together with excipients useful to formulate the NSAID into a dosage form and also excipients useful to ensure rapid disintegration which can be produced by a simple and cost effective large scale manufacturing processes. When it is desired to provide the formulation as a tablet, pharmaceutically acceptable excipients must be chosen for combination with the NSAID, with which the NSAID is compatible and with which it can form a dosage form which is stable on storage. Desirably, tablets having a satisfactory hardness but also being able to disintegrate rapidly when added to a liquid drink may be prepared. A further issue to overcome in a formulation adapted for oral consumption is the poor taste of ibuprofen and other NSAIDs.

We have now found that when an acidic or basic component of an effervescent couple is mixed an NSAID and the mixture is heated until the NSAID is molten and then cooled and formed into granules or when an acidic or basic component of an effervescent couple is mixed with a molten NSAID and the mixture cooled and formed into granules, a storage-stable formulation having advantageous dissolution properties is provided. A drink product produced using these granules typically has an acceptable taste.

Accordingly, the present invention provides an effervescent formulation comprising a low-melting non-steroidal anti-inflammatory drug (NSAID) and an effervescent couple comprising:
(a) a granular composition comprising a plurality of solidified melt granules of the NSAID with either an acidic or a basic component of the effervescent couple uniformly dispersed therein; and
(b) an extra-granular composition comprising the other of the basic or acidic component of the effervescent couple.

In a preferred aspect, the present invention provides an effervescent formulation comprising a low melting non-steroidal anti-inflammatory drug and an effervescent couple comprising:
(a) a granular composition comprising a plurality of solidified melt granules of the NSAID with an acidic component of the effervescent couple uniformly dispersed therein; and
(b) an extra-granular composition comprising a basic component of the effervescent couple.

Alternatively, a basic component of the effervescent couple may be contained within the granular composition and an acidic component of the effervescent couple may be contained within the extra-granular composition.

Formulations according to the present invention have been found to be stable on storage and to have advantageous dissolution properties. In addition, they may be prepared by a simple cost-efficient manufacturing process on a large scale. The formulation may be tabletted without sticking or capping during the tabletting process to provide a dosage form having suitable hardness properties combined with advantageous disintegration properties. Furthermore, the poor taste is significantly improved in the resulting drink product. Additionally, the separation of the effervescent couple by this process enables packaging materials with lower moisture barrier properties to be employed resulting in further cost advantages.

The melt granules may be formed by combining the NSAID and the acidic or basic component of the effervescent couple in the solid state and then heating until at least the NSAID is molten. A fluid liquid is formed. Alternatively, the acidic or basic component of the effervescent couple may be added to molten NSAID. In both cases, the liquid melt is cooled until a solidified melt is formed. In both methods, the acidic or basic component of the effervescent couple is intimately mixed with the NSAID in the granular composition and forms a uniform solid dispersion in the solidified NSAID melt. Thus, as used herein, "solidified melt granules" means granules comprising a combination of the NSAID which has been melted and solidified and an acidic or basic component of an effervescent couple uniformly dispersed therein.

Preferably, the solidified melt granules comprise an acidic component of an effervescent couple.

It has been found that the crystalline form of the NSAID changes on melting and then cooling the NSAID. For example, the crystals become smaller and the surface area of the NSAID in the melt granule is increased compared to that of conventional crystals of the NSAID. In addition, on cooling, the changes to the crystalline structure lead to a more porous granule.

The crystalline structure of the melt granules formed from solidifying fully melted NSAID differs from the crystalline structure where the NSAID is only partially melted. In the case of partial melting, the crystalline structure of the melted NSAID is interrupted by the non-melted NSAID, thus providing that the NSAID does not have a single crystalline structure. The NSAID is preferably fully melted so that on cooling, a single continuous phase of the NSAID is formed. That is to say the crystalline structure of the NSAID is not interrupted by another crystalline NSAID structure. In preferred compositions according to the present invention, the NSAID is present in a single crystalline state, accordingly the NSAID continuous phase comprises a single crystalline phase of the NSAID. The NSAID suitably forms a continuous phase in the granule with the acidic component of the effervescent couple uniformly dispersed therein. The melt granules are thus in the form of a solidified melt of NSAID. The melt granules preferably comprise said NSAID as a continuous phase.

The granules produced on cooling the molten drug are of a suitable size for tabletting, preferably in a standard large scale tabletting machine. The melt granules in the granular composition preferably have a mean particle size in the range 10-2000µm, more preferably 50-1000µm and most preferably 100-400µm. Valuable results are achieved when the bulk density of the melt granules is in the range 0.1-1gml⁻¹, more preferably 0.3-0.6gml⁻¹. Further preferred properties are obtained when the tapped density is in the range 0.3-0.7gml⁻¹ (more preferably 0.4-0.6 gml⁻¹). Further, preferably the melt granules have a porosity of 0.5-2.0 g/ml.

The invention allows the formulation of any relatively low melting NSAID into an acceptably tasting, readily disintegrating composition. It is generally envisaged that the melting point of such compounds will be low enough to allow the melting thereof using standard equipment. It is also important that there is not a deleterious effect on the NSAID itself or the ingredients incorporated in the molten NSAID, for example the acid. Thus, typical melting points of the low melting NSAIDs would be expected to fall within the range 30-300°C. Preferred NSAIDs have lower melting points so that the melting process does not use significant amounts of energy, which thus has an effect on production costs. A favoured class of compounds are the 2-arylpropionic acids which are generally substantially insoluble and have poor taste properties. Preferred NSAIDS have melting points in the range 30-200°C (such as racemic naproxen, melting point 156°C), more preferably 30-150°C, further preferably 40-120°C (such as racemic flurbiprofen, melting point 114°C), most preferably 40-100°C (such as racemic ibuprofen (melting point 75-77°C), S(+)-ibuprofen (melting point 52-54°C) and racemic ketoprofen (melting point 96°C)). Preferred low-melting NSAIDs are naproxen, ketoprofen, flurbiprofen, ibuprofen or enantiomers thereof. Preferably, the NSAID is in the form of a racemic mixture or in the form of a single enantiomer (especially the S(+)-enantiomers) thereof. Salts of racemic NSAIDS and enantiomers thereof may also be used. Preferred salts are the sodium salts, potassium salts and lysine salts.

The invention is especially adapted for an ibuprofen medicament. The term "ibuprofen medicament" preferably includes racemic ibuprofen and S(+)-ibuprofen and their sodium, potassium and lysine salts which have low melting points and a very poor after-taste in the mouth and throat. Most advantageous results are obtained with racemic ibuprofen which has a high dosage combined with poor solubility properties.

The proportion of NSAID in the melt granules will depend on the dose desired for therapeutic effect. Low dose drugs, such as flurbiprofen and ketoprofen may form as little as 1% by weight if a relatively large dosage form is required. However, particular advantages of the present invention are obtained by allowing a reduction in the number and/or amount of excipients. Accordingly the NSAID may form up to 95% of the melt granules. Accordingly, it is generally envisaged that the NSAID will generally form 10-95% w/w of the melt granule, preferably 5-80%, more preferably 15-50% of the melt granule. A preferred feature of the invention is that low-melting, high dose NSAIDs, such as ibuprofen, can be formulated into smaller effervescent dosage forms. Accordingly, the NSAID will suitably form 10-80% w/w of the melt granules, preferably 20-70% and further preferably 25-60% w/w of the melt granules.

In order to accommodate the effervescent couple, the NSAID will usually form less than 80% w/w of the formulation, for example 1-80% w/w, suitably 5-70% w/w, preferably 10-60% w/w, more preferably 20-55% w/w and most preferably 30-50% w/w of the formulation.

The effervescent couple comprises an acidic component and a basic component which are adapted to react in the presence of water to produce carbon dioxide. The gas formed causes the formulation to disintegrate quickly. In a formulation according to the present invention, the acidic or basic component is enveloped within the NSAID, preferably within the NSAID as the continuous phase, and so is prevented from premature reaction with the basic or acidic part of the effervescent couple in the extra-granular composition. Preferably, the acidic component of the effervescent couple is enveloped within the NSAID and the basic part of the effervescent couple is in the extra-granular composition.

The ratio of the effervescent couple to said non-steroidal anti-inflammatory drug is generally in the range 10:1 to 1:10 parts by weight. Suitably the formulation may comprise the effervescent couple in an amount of 10-90% w/w, preferably 20-90% w/w, more preferably 30-80% w/w and most preferably 40-75% w/w. The ratio of the acidic to the basic component in the effervescent couple is suitably 1:3 to 3:1 parts by weight, preferably 1:2 to 2:1, most preferably 1:1 to 1:2 parts by weight.

The acidic component of the effervescent couple is preferably contained in the granular composition. Suitably, it is an edible organic acid. Preferred examples include citric, tartaric, malic, fumaric, ascorbic and adipic acids. Suitably the melt granules comprise 3-80% acidic component, preferably 10-80%, more preferably 20-60% w/w. Preferably, the formulation comprises 10-70% acidic component, more preferably 20-40% w/w and most preferably 15-35% w/w. If the basic component of the effervescent couple is contained in the solidified melt granules, the percentage of the basic component within the granules is suitably 5 to 90%, preferably 10-80%, more preferably 20-60% w/w.

The ratio of NSAID to acid will depend on the proportion of the NSAID in the dosage form. Thus, depending on the dosage of the drug, it can be expected to fall in the range 20:1 to 1:20, conveniently 10:1 to 1:10. For relatively high dose drugs such as ibuprofen and naproxen, the ratio of NSAID to acidic component may be in the range 1:2 to 5:1, more preferably 1:1 to 3:1, most preferably 1:1 to 2:1. For relatively low dose drugs such as flurbiprofen and ketoprofen, the ratio of NSAID to acidic component may suitably be 1:1 to 1:20, preferably 1:5 to 1:15.

The acidic or basic component is most commonly insoluble in the ibuprofen melt and a dispersion of the solid acidic or basic component within the liquid melt is produced. The dispersion is mixed so that the acidic or basic component is uniformly or homogeneously combined with the melted NSAID. A uniform mixture is thus produced. As the mixture cools, it becomes more viscous. The mixture is allowed to cool by methods hereinafter discussed until a solid is produced. The melt granules may be formed before, during or after the NSAID solidifies.

Suitable basic components include alkali metal salts (such as sodium or potassium salts) of carbonates. Preferred basic components include sodium carbonate, sodium bicarbonate, potassium carbonate and/or potassium bicarbonate, especially sodium bicarbonate. Suitably the extra-granular composition comprises the basic component in an amount of 80-100% w/w, preferably 90-100% w/w. Preferably, the formulation comprises 10-80% w/w basic component, preferably 30-50%, more preferably 25-40% w/w. If the acidic component of the effervescent couple is contained in the extra-granular composition, the percentage of the acidic component within the extra-granular composition is suitably 75-100%, preferably 85-99% w/w

The ratio of NSAID to basic component will depend on the proportion of the NSAID in the dosage form. Thus, depending on the drug dose, it can be expected to fall in the range 20:1 to 1:20, conveniently 10:1 to 1:10 parts by weight. For relatively high dose drugs, such as ibuprofen and naproxen, the ratio of NSAID to basic component is in the range 1:2 to 5:1, more preferably 1:1 to 3:1, most preferably 1:1 to 2:1. For relatively low dose drugs, such as flurbiprofen and ketoprofen, the ratio of NSAID to basic component is suitably 1:1 to 1:20 parts by weight, preferably 1:5 to 1:15 parts by weight.

The extra-granular composition comprises the ingredients incorporated in the formulation which are not contained in the solidified melt granules. The ingredients of the extra-granular composition may be mixed with the melt granules simultaneously or at sequential stages in the process to prepare the formulation. A particular advantage of the present invention is preferably that all the ingredients of the extra-granular component are combined with the melt granules at a single stage in the manufacturing process. Also, it is preferred that at this stage, the ingredients in the extra-granular component are combined sequentially with the melt granules. The formulation comprises a uniform mixture of melt granules and extra-granular component. The extra-granular component is suitably distributed evenly throughout the formulation.

The extra-granular composition may also comprise flow aids such as silicon dioxide and talc.

Silicon dioxide is insoluble in water and suitably has a surface area greater than 50 m²g⁻¹, more preferably greater than 100 m²g⁻¹, especially in the range 150-250 m²g⁻¹. Most preferably the silicon dioxide is colloidal silicon dioxide (especially having a mean particle size less than 50nm such as 5-40nm), most preferably anhydrous colloidal silicon dioxide. The tapped density of the silicon dioxide is preferably in the range 0.01-0.2gcm⁻². The silicon dioxide may be incorporated in the composition to an extent of 0.05-5.0% w/w, preferably 0.1-3% by weight, more preferably 0.2-1% w/w of the composition. Silicon dioxide is a preferred component of the extra-granular composition.

The formulation may also comprise a disintegrant. The addition of a disintegrant further improves the rate at which the tablet breaks up when added to a liquid medium. Examples of disintegrants include one or more of starch and modified starch (such as wheat starch, maize starch, potato starch), sodium starch glycolate, low-substituted hydroxypropyl cellulose, alginic acid, cross-linked polyvinylpyrrolidone, magnesium aluminium silicate, bentonite and croscarmellose sodium. Preferred disintegrants are those which swell on the action of water, thus causing the ingredients in the tablet to be pushed apart and out into the aqueous disintegration medium. Preferred examples of disintegrants are croscarmellose sodium and/or sodium starch glycolate, especially croscarmellose sodium. The disintegrant is present at an effective disintegrating amount, for example up to 50% w/w of the formulation (e.g. 1-50% w/w), more preferably 1-25% w/w, further preferably 2-20% w/w and most preferably 2-15% w/w of the formulation.

The disintegrant is an optional ingredient of the melt granules and/or the extra-granular composition. The disintegrant will suitably form 0.1-25% w/w of the melt granules, preferably 3-15% w/w and most preferably 4-10% w/w of the melt granules. The disintegrant may be present in the extra-granular composition in an amount of 0.1 to 25% w/w, preferably 1-15%, more preferably 2-10% w/w.

The ratio of NSAID to disintegrant will depend on the proportion of the NSAID in the dosage form. Thus, depending on the dosage of the drug, it can be expected to fall in the range 20:1 to 1:20, conveniently 10:1 to 1:10. For relatively high dose drugs, such as ibuprofen and naproxen, the ratio of NSAID to disintegrant may be in the range 20:1 to 2:1, preferably 10:1 to 5:1 parts by weight. For relatively low dose drugs, such as flurbiprofen and ketoprofen, the ratio of NSAID to disintegrant is suitably 10:1 to 1:10, for example 5:1, preferably 1:1 to 1:5 parts by weight.

Although not necessary for the production of compositions according to the present invention, if desired, the dosage form may further comprise a diluent. The diluent, may be water-soluble or water-insoluble. Suitable water-soluble diluent materials include the sugar alcohols (such as xylitol, sorbitol, mannitol, erythritol), sugars (such as sucrose, fructose, lactose, dextrose), cyclodextrin, maltodextrin and salts of organic acids (e.g. sodium citrate and potassium citrate). Lactose, sodium citrate and potassium citrate are particularly preferred water-soluble diluents. Suitable water-insoluble diluent materials include cellulose derivatives (such as microcrystalline cellulose) starch and derivatives thereof (such as pre-gelatinised starch), dicalcium phosphate, tricalcium phosphate, calcium sulphate, calcium carbonate. Microcrystalline cellulose and dicalcium phosphate are preferred water insoluble diluents.

The diluent, if used, is typically present in an amount of from 0.1 to 60%w/w of the formulation.

The diluent may preferably include a basic ingredient such as an alkali metal salt for example an alkali metal carbonate, bicarbonate or citrate, present to an extent of up to 50% by weight (e.g. in the range 1-50% by weight), preferably up to 40% by weight (e.g. in the range 1-40% by weight) of the composition (more preferably 2-35% w/w and most preferably 10-20% w/w). Preferably, the alkali metal salt is sodium or potassium. Further preferably, the salt is a citrate, carbonate or bicarbonate salt of sodium or potassium, more preferably sodium bicarbonate or citrate. The ratio of NSAID (especially ibuprofen medicament) to alkali metal salt may be in the range 100:1 to 1:1 parts by weight, preferably 5:1 to 1:1 parts by weight. Preferably, the alkali metal salt is incorporated in any amount up to an equimolar amount with respect to the NSAID (e.g. ibuprofen). Conveniently, a sub-molar amount of alkali metal salt is incorporated. Thus the alkali metal compound may form up to 100% w/w of the NSAID, preferably 50% w/w, more preferably up to 10% w/w, of the NSAID. In a preferred compressed tablet according to the present invention, the NSAID (especially an ibuprofen medicament) is in admixture with the alkali metal salt. The alkali metal salt is preferably incorporated in the melt granules.

In a formulation adapted to disperse in water prior to administration, the level of diluent may be quite high, for example up to 50% (such as 0-50% w/w, preferably 0-40% w/w) by weight of the formulation in order to achieve the desired dispersing properties. Preferably, the diluent does not form greater than 25% by weight of the formulation (e.g. 0-25% w/w), as it adds to the costs of the composition and to production costs. Thus, to minimise costs it may be preferred that the diluent is added to the formulation in an amount of 0-20% by weight of the formulation, more preferably 0-10% w/w. If present, it may be preferably used to an extent of 0.1-25% by weight of the formulation, more preferably 0.1-20% w/w, further preferably 0.1-10% w/w and most preferably 1-5% by weight of the formulation.

The diluent may be contained in the melt granule or may be part of the extra-granular composition or may be incorporated as desired in both components. If desired, the diluent may preferably be added in an amount up to 30% w/w of the extra-granular component (i.e. 0.1-30% w/w, although to minimise the size and cost of the dosage form, it is desired to include a minimum amount of such additional excipients. Accordingly, if employed, the diluent may suitably be included in the extra-granular composition in the range up to 30% w/w (i.e. 0.1-30%), preferably 0.1-15% w/w, more preferably 0.1-10% w/w. As discussed hereinabove, the diluent may be present in the melt granules, for example in an amount of 0-30% w/w (such as 0.1-30%) by weight of the melt granules. If present, the diluent conveniently forms 1-20%, more preferably 1-10% w/w of the melt granules.

The formulation may also include a surfactant, in the amount appropriate to the properties of the surfactant, preferably 0.05-10% by weight of the formulation. The surfactant may be included in the melt granules and/or the extra-granular composition. Preferred surfactants are sodium lauryl sulphate, poloxamer, hydrogenated castor oil and derivatives thereof, polyoxyethylene surfactants (including polyoxyethylene oils, fatty acid esters, including stearates) and sorbitan esters. They may be used to an extent of 0.05-5% w/w, preferably 0.1-3% w/w, more preferably 0.2-2% w/w) of either or both the melt granules and the extra-granular composition.

Optionally a lubricant may be incorporated in the extra-granular composition for mixing with the melt granules. Conventional lubricants for NSAIDS may be used for example stearic acid, sodium lauryl sulphate, polyethylene glycol, hydrogenated vegetable oil, sodium stearyl fumarate, magnesium stearate or calcium stearate. These may be present in an amount from 0.05-5% by weight, preferably 0.1-2% by weight of the formulation. Anti-adherents such as talc, may further be included in an amount of up to 4% by weight of the dosage form, for example 0.5-2% by weight of the dosage form, preferably as part of the extra-granular component.

Other conventional tabletting excipients known to the person skilled in the art may be incorporated in the compressed tablet composition according to the present invention as desired, although it will be appreciated that a prime advantage of the present invention is that the number of excipients necessary to achieve a quickly disintegrating tablet with good dissolution characteristics is minimal.

The acidic or basic component of the effervescent couple (preferably the acidic component) may be the sole ingredient incorporated within the NSAID (preferably ibuprofen) melt granules or it may be combined with a diluent and optionally a surfactant and other tabletting excipients. Accordingly, in one preferred embodiment, the granules may comprise greater than 90% w/w of the NSAID and acidic or basic component of the couple (i.e. 90-100% w/w). Preferred melt granules comprise an NSAID (preferably ibuprofen), an acid, a disintegrant and optionally a surfactant and/or a diluent. In a further preferred embodiment, the granules comprise greater than 90% w/w (i.e. 95-100% w/w) of the combination of NSAID, acidic component and disintegrant. Further preferably, the granules consist essentially of (98-100% w/w) of the combination of NSAID, acidic component, a disintegrant. The optional diluent and the optional surfactant may be combined with the acidic component, the disintegrant and the drug in fully molten form. Further preferred melt granules consist essentially of an NSAID (preferably ibuprofen), an acid, a disintegrant and a surfactant. A further preferred granular component consists essentially of an NSAID (preferably ibuprofen), a disintegrant, a surfactant and a diluent. Preferably the NSAID is ibuprofen.

In a preferred formulation according to the present invention the melt granules comprise:
(a) 5-40% w/w non-steroidal anti-inflammatory drug;
(b) 20-80% w/w acidic component; and
(c) 0.1-15% w/w disintegrant.

An advantageous formulation (especially a tablet formulation) according to the present invention comprises an extra-granular composition comprising a basic component of an effervescent couple, silicon dioxide and a lubricant and/or a surfactant. This may form an intimate admixture with said melt granules prior to filling into a unit dose (e.g. a sachet or capsule) or compression into a tablet. Preferably, the extra-granular component, excluding the basic component, consists essentially of silicon dioxide and a lubricant in a ratio of one part by weight silicon dioxide to 0.5-5 parts by weight lubricant, more preferably 0.5-2 parts by weight lubricant.

The formulation preferably comprises a combination of the melt granules with the extra-granular composition comprising silicon dioxide and optionally a lubricant. This combination may take the form of a uniform or homogeneous blend capable of being mixed with other ingredients as desired and formed into a unit dose. The unit dose may be swallowed and thus release the ibuprofen medicament in the stomach or gastro-intestinal tract or dispersed in water prior to ingestion. Preferably the formulation is in the form of a powder mixture, such as granules or a tablet, which is adapted to be added to water prior to administration.

In another preferred aspect of the invention, the extra-granular component comprises at least one of silicon dioxide, stearic acid or a salt thereof, a surfactant and a diluent.

A preferred formulation according to the present invention comprises:
(a) 10-25% w/w ibuprofen;
(b) 25-40% w/w acidic component;
(c) 0.5-10% w/w croscarmellose sodium;
(d) 25-50% w/w sodium bicarbonate; and
(e) 0.05%-5% w/w surfactant.

Another preferred formulation of the invention comprises:
(a) 10 - 90% w/w melt granules; and
(b) 90 - 10% w/w extra-granular composition.

Another preferred formulation of the invention comprises:
(a) 40 - 80% w/w melt granules consisting essentially of said NSAID, said acidic component and a disintegrant;
(b) 20 - 60% w/w extra-granular composition comprising a base in an amount greater than 90% of said extra-granular composition.

The compressed tablet composition of the present invention may, if desired, include other compatible pharmacologically active ingredients and/or enhancing agents. Thus, for example, the dosage form may include any other ingredient commonly used in a composition useful to treat pain, inflammation and/or fever, for example caffeine or another xanthine derivative, another analgesic, for example codeine, a skeletal muscle relaxant: an antihistamine (e.g. acrivastine, astemizole, azatadine, azelastine, bromodiphenhydramine, brompheniramine, carbinoxamine, cetirizine, chlorpheniramine, cyproheptadine, dexbromopheniramine, dexchloropheniramine, diphenhydramine, ebastine, ketotifen, lodoxamide, loratidine, levocabastine, mequitazine, oxatomide, phenindamine, phenyltoloxamine, pyrilamine, setastine, tazifylline, temelastine, terfenidine, tripelennamine or triprolidine (preferably non-sedating antihistamines are employed)); a decongestant (e.g. pseudoephedrine, phenylpropanolamine and phenylephrine); a cough suppressant (e.g. caramiphen, codeine or dextromethorpan); an anti-nauseant (e.g. domperidone) and/or an expectorant (e.g. guaifenesin, potassium citrate, potassium guaiacolsuphonate, potassium sulphate and terpin hydrate).

Such extra active ingredients and/or enhancing agents may be incorporated in the melt granules or in the extra-granular composition in appropriate dosage amounts for the desired therapeutic effect. Reference may be made to MIMS and the Physicians Desk Reference for guidelines as to a suitable dosage. It is generally expected that such other active ingredients will form 0-50% w/w of the formulation, for example 5-25% w/w.

NSAIDs such as Ibuprofen and its derivatives are primarily anti-inflammatory, analgesic and anti-pyretic agents but have also been proposed for other therapeutic uses, including the treatment of periodontal bone loss, pruritus and Alzheimer's disease. The dosage forms of the present invention are therefore indicated for use in the treatment of all therapeutic uses for which ibuprofen is effective, including rheumatoid arthritis, osteoarthritis, ankylosing spondylitis, seronegative arthropathies, periarticular disorders and soft tissue injuries. They may also be used in the treatment of postoperative pain, postpartum pain, dental pain, dysmenorrhoea, headache, migraine, rheumatic pain, muscular pain, backache, neuralgia and/or musculoskeletal pain or the pain or discomfort associated with the following: respiratory infections, colds or influenza, gout or morning stiffness.

Accordingly, the present invention provides compositions for use in the treatment of pain and/or inflammation and/or fever, in particular, for use in the treatment of postoperative pain, postpartum pain, dental pain, dysmenorrhoea, headache, migraine, rheumatic pain, muscular pain, backache, neuralgia and/or musculoskeletal pain or the pain or discomfort associated with the following: respiratory infections, colds or influenza, gout or morning stiffness.

Furthermore, the invention also provides a method of treating pain and/or inflammation and/or fever comprising the administration of a composition according to the present invention to a mammal in need thereof.

The formulations of the present invention can be used in the manufacture of a medicament for treating pain and/or inflammation and/or fever, in particular, for treating postoperative pain, postpartum pain, dental pain, dysmenorrhoea, headache, migraine, rheumatic pain, muscular pain, backache, neuralgia and/or musculoskeletal pain or the pain or discomfort associated with the following: respiratory infections, colds or influenza, gout or morning stiffness.

Unit dosages for effective therapy are known to those skilled in the art for each NSAID. For example, they may comprise the NSAID to an extent of 5mg, 10mg, 12.5mg, 25mg, 50mg, 100mg, 150mg, 200mg, 250mg, 300mg, 350mg, 400mg, 500mg, 600mg and 800mg. Where derivatives are employed, normally the precise unit dosages are chosen to give the equivalent NSAID doses given above. For the treatments described herein the maximum daily dose of ibuprofen is generally 3200 mg. A single unit daily dose may be 100 mg. Preferred unit doses are in the range 100-400 mg, more preferably 100-300 mg and especially 200 mg ibuprofen. The maximum daily dose of flurbiprofen is generally 300 mg. A single unit dose may be 12.5 mg. Preferred unit doses are in the range 12.5-150 mg, more preferably 25-100 mg and especially 50 mg flurbiprofen. The maximum daily dose of naproxen is generally 1500 mg. A single unit daily dose may be 125 mg. Preferred unit doses are in the range 220-750 mg, more preferably 220-500 mg and especially 220-250 mg naproxen. The maximum daily dose of ketoprofen is generally 200 mg. A single unit dose may be 25 mg. Preferred unit doses are in the range 25-100 mg, more preferably 25-75 mg and especially 50 mg ketoprofen.

In a further aspect, the present invention provides a process for the preparation of a formulation according to the invention, comprising:
(a)
   (i) combining an acidic or basic component of an effervescent couple with the NSAID in the solid state and heating the mixture until the NSAID is molten; or
   (ii) heating the NSAID until it is molten and combining the acidic or basic component of an effervescent couple with the molten NSAID to form a mixture thereof;
(b) forming the mixture into solidified melt granules;
(c) combining said solidified melt granules with an extra-granular composition comprising the other of the basic or acidic component of the effervescent couple.

A preferred process of the invention comprises:
a) heating the NSAID until it is molten and then combining the molten NSAID with an acidic component of an effervescent couple to form a mixture thereof;
b) forming said mixture into solidified melt granules with cooling;
c) combining said solidified melt granules with an extra-granular composition comprising a basic component of an effervescent couple.

Preferably, the combined melt granules/extra-granular composition mixture from step (c) is then formed into a unit dose.

When the acidic or basic component of the effervescent couple is combined with a molten NSAID the acidic or basic component is typically in the solid state. When the acidic or basic component is mixed with the NSAID before the NSAID is melted, the acidic or basic component typically does not melt on heating to the temperature required to melt the NSAID. Thus, a suspension or dispersion of the acidic or basic component in the molten drug will be formed.

At room temperature, the NSAIDs used in accordance with the present invention are solid. Thus, it is necessary to heat the NSAID to a temperature above its melting point so that it becomes molten. In a process according to certain embodiments of the present invention, the acidic component (or the basic component) of the effervescent couple and the solid drug are mixed uniformly in the solid state prior to melting the drug. In alternative embodiments, the NSAID is melted and the acidic component (or the basic component) is added to the melt.

Under pressurised conditions, the drug may be melted at a temperature below its normal melting point. Melting may be carried out according to known methods, including for example, heating in a vessel to a temperature above the melting point of the NSAID or by extrusion in a heated extruder. The maximum temperature is determined by the stability of the molten drug and ingredients combined therewith. The drug may be heated to any convenient temperature. Generally, the higher the temperature, the more quickly the drug will melt although this must be balanced by the energy input required to heat the drug. For highest efficiency, it is generally envisaged that the NSAID will be heated to not more than 50°C, preferably 1-25°C and more preferably 5-20°C, above its melting point to keep energy costs to a minimum. A preferred heating range is 30-180°C, more preferably 35-140°C and further preferably 40-120°C.

If the NSAID is extruded, generally the extruder is typically heated to a predetermined temperature. In addition, the work on the NSAID by the screw configuration in the extruder will also contribute to melting the NSAID thereby reducing its external applied temperature requirement. Accordingly the extruder barrel may be heated to a temperature less than the melting point of the NSAID. For example, the normal melting point of racemic ibuprofen is 75-77°C, however under conditions of force/pressure (such as may be encountered in an extruder or similar processing device), the external applied heat necessary to melt the ibuprofen may be reduced significantly through the mechanical heat generated by the intense mixing action within the extruder. It is generally envisaged that the extruder will be heated to a temperature not less than 25°C below the melting point of the drug, preferably in the range from 20°C below the melting point of the drug to 50°C above the melting point of the drug, more preferably from 15°C below the melting point of the drug to 25°C above its melting point and most preferably to a temperature in the range of 10°C on each side of the melting point of the drug. Some extruders allow different zones to be heated to different temperatures in the extruder. These temperatures can be chosen as desired to ensure that the NSAID is melted, preferably fully melted, in stage (a). Preferably, the drug and acidic component are heated to a temperature in the range 80-130°C, more preferably 100-120°C to melt said drug. When the NSAID is ibuprofen it may conveniently be heated in the range 50-130°C, more preferably 60-100°C. When heated by conventional heating means such as a water or steam bath it is preferably heated in the range 75-90°C, more preferably 75-85°C. The ibuprofen may also be heated and subjected to conditions of force, such as by heat-extruding the ibuprofen, for example in a twin-screw extruder. The temperature of the ibuprofen in the extruder barrel is preferably in the range 66-96°C, preferably 70-82°C. The acidic or basic component will be dispersed throughout the melt by conventional blending and/or stirring techniques. A molten mixture of the acidic or basic component within the melted drug is thus formed.

Preferably, a disintegrant is also combined with said drug in molten form. In a further preferred aspect, at least one of a surfactant and a diluent is combined with said drug in molten form. These excipients may be added prior to heating of the NSAID or when the NSAID is in a molten form.

Conveniently, heating and blending (e.g. stirring, agitating, kneading or extruding the NSAID) occurs at the same time. The above-mentioned process may be carried out in a number of ways. The manner in which these additional excipients are combined with the drug will depend on conditions, including the drug used and the dosage thereof, the temperature to which the drug is heated, the amount of effervescent couple and any other excipients used, the quantity of ingredients being processed and will be within the knowledge of the person skilled in the art. In one method, the NSAID is heated in a suitable vessel until molten. The acidic or basic component may then be added to the molten mass and thoroughly combined therewith to form a homogeneous mixture. Optional extra excipients may also be blended into the molten NSAID simultaneously or sequentially.

In a further process, the non-steroidal anti-inflammatory drug may be combined with the acidic or basic component and optional excipients, e.g. a diluent, and then heated together until said non-steroidal anti-inflammatory drug is molten. In yet a further process the NSAID and acidic or basic component are combined in the solid state and heated together until said NSAID is molten and any further desired tabletting excipients uniformly blended with the mixture. It may also be desired to combine the NSAID with at least one additional excipient in the solid state, then heat until said NSAID is molten and then add the acidic or basic component together with any additional desired excipients.

In another method, the NSAID and acidic or basic component and any optional granular ingredients are fed into an extruder type system (preferably having first been combined by blending together). The materials are heated and mixed by kneading by the screw(s) within the extruder until the NSAID is molten and a uniform mixture of the components is produced. Preferably, the acidic or basic component and drug are melt extruded. Further preferably, the NSAID and the acidic or basic component are extruded in a twin screw extruder.

The molten mixture may then be discharged into an appropriate cooling system, for example a cooled belt which may continuously rotate and deliver the cooled melt to a comminuting device such as a scraper bar and/or a mill.

The molten mixture is formed into solidified melt granules. The melt is allowed to solidify in any manner convenient. In some cases, the melt granules may be formed whilst the drug is in the molten state (i.e. fully molten or partially molten) and then allowed to cool to form the solidified melt granules. This may be carried out, for example in a melt-extrusion process, for example the extrudate may be chopped into pellets as it passes out of the extruder and the pellets cooled in an appropriate manner, such as a cooling belt. In a further method, after heating or heat-extrusion the NSAID and acidic component may be cooled by feeding either to a spray tower dryer or to a spray granulator in which the molten mass is sprayed into the path of a stream of cold air to form droplets and the dried solid mass collected.

Generally, it is expected that the molten mixture will be cooled to a temperature below the melting point of the drug before being formed into granules. This includes both rapid cooling and slow cooling. Preferably the molten drug mixture is cooled rapidly. For example, the molten NSAID may be allowed to cool overnight at ordinary temperatures or in a cooled vessel. Preferably, the molten drug mixture is cooled rapidly. For example, the molten NSAID may solidify in 5 minutes or less, preferably in 3 minutes or less, more preferably in 1 minute or less (e.g. 0 to 60 seconds), preferably 50 seconds or less (e.g. 1 to 50 seconds), more preferably 1 to 40 seconds and most preferably 1 to 30 seconds.

The molten NSAID may be poured onto cooling trays which may be static or continuously moving. Static trays may be placed in cooling cabinets. Moving trays or belts may have additional cooling means, such as cooled water. The cooled melt forms a solid and may be scraped off the belt or collected as it falls off one end of a continuously moving belt. Preferably, the uniform mixture of step (a) is cooled to the solid state before being formed into granules. The molten drug mixture from step (a) may be discharged into an appropriate cooling system, for example a tray or other vessel, and allowed to cool at room temperature. In another process, the tray or vessel may be cooled (e.g. water-cooled). In order to maintain a uniform mixture of the acidic component within the drug, it may be necessary to agitate or stir the mixture during cooling. Cooling may also occur by cooling in a stream of cooled air. The molten drug mixture may also be cooled by passing the molten mixture onto a moving cooling belt, preferably a continuously rotating cooling belt. Preferably, the belt is cooled by water. The water may be applied to the underside of the belt along its length or partially along its length as desired and according to the length of the belt, the quantity of molten drug mixture and the speed of the belt. It is especially preferred to cool the molten drug mixture at least initially by cooling means, for example until it has started to solidify. Advantageously, the belt is water-cooled along substantially the whole of its length and it is of minimum length required (e.g. 3-7m) to allow it to cool to the solid state.

The solidified melt incorporating the acidic or basic component may be formed into granules by a plurality of methods. For example, it may be pulverised or comminuted into granules. It may be milled and/or sieved. If it is cooled on a moving belt, the cooled melt may be delivered to a comminuting device such as a scraper bar and/or mill. It may also be passed through a spray device such as a spray tower or spray granulator in which the molten material is sprayed from an orifice into a stream of cooled air, allowed to congeal/solidify and then collected. If the molten NSAID is extruded, the extrudate may be cooled and then broken into conveniently sized pieces, followed by milling and or sieving. Alternatively, the extrudate may be extruded through holes and chopped into suitably sized granules for tabletting.

Preferably, the molten drug mixture is formed into a ribbon or ribbons and is cooled on a water-cooled belt. The solidified ribbons may be milled into granules. The granular composition may be sieved to ensure that the melt granules are of the appropriate size for efficient tabletting.

The solidified melt granules are combined with an extra-granular component comprising a basic or acidic component of an effervescent couple. This may be achieved by conventional mixing and blending techniques. Examples of apparatus that may be used to facilitate this process are: Ribbon Blender, IBC Blender, V-Bender and Plough Benders.

The melt granules are combined thoroughly so as to form a uniform mixture of ingredients. The extra-granular composition should be distributed evenly through the formulation to maximise the effervescent reaction between the acidic and basic components of the couple.

The combined melt granules and extra granular composition may be formed into a unit dose. Examples include filling of the loose powder mixture into a sachet or a capsule or compressing it into a tablet. Tablets are the preferred unit dosage form according to the invention. They may be swallowed or they may be chewed. It has unexpectedly been found that the taste of the NSAID has been substantially masked which allows the dosage form to be maintained in the oral cavity for a period of time whilst the formulation is swallowed.

The formulations of the present invention, for example tablet formulations, may optionally be coated with a film coat, for example based on a conventional cellulose polymer such as hydroxypropylmethyl cellulose, or a conventional sugar coat, for example based on sucrose or lactose.

In a preferred aspect, the extra-granular composition comprises at least one of silicon dioxide, stearic acid or a salt thereof, a surfactant and diluent.

In a preferred aspect, said NSAID comprises Ibuprofen.

In a further aspect of the invention, there is provided a granular composition comprising a plurality of solidified melt granules of a low-melting non-steroidal anti-inflammatory drug (preferably having a melting point in the range 30-300°C) characterised in that it further comprises an edible organic acid. A preferred granular composition comprises a disintegrant, especially in an amount of 2-8% w/w of the granular composition.

The present invention also provides a process for the preparation of such a granular composition, comprising:
(a)
   (i) combining the acidic component of an effervescent couple with the NSAID in the solid state and heating the mixture until the NSAID is molten; or
   (ii) heating the NSAID until it is molten and combining the acidic component of an effervescent couple with the molten NSAID to form a mixture thereof;
(b) forming a uniform mixture; and
(c) forming said mixture into solidified melt granules with cooling.

A preferred process for preparing a granular composition comprises:
(a) heating the NSAID until it is molten and then combining the molten NSAID with an acidic component of an effervescent couple to form a mixture thereof;
(b) forming a uniform mixture; and
(c) forming said mixture into solidified melt granules.

Other preferred features of the process for producing the granular composition are as described above for the preparation of the formulations of the invention.

The invention is illustrated by the following non-limiting Examples. In the Examples, the racemic ibuprofen and racemic flurbiprofen is available from BASF Pharma, USA; colloidal silicon dioxide (also known as colloidal silica) is available from Degussa, Frankfurt, DE under the trade name Aerosil 200;Croscarmellose sodium is available from the FMC Corporation Brussels, BE under the tradename Ac-Di-Sol; and sodium starch glycolate is available from Edward Mendell, Reigate, GB under the tradename Explotab and Poloxamer is available from BASF, DE under the trade name Pluronic F68.

### Dissolution Measurement

The dissolution may be measured using the dissolution method described in the US Pharmacopoeia Vol. 23, page 1791, Apparatus 2 using paddles at 50 rpm and a phosphate buffer (selected at pH 7.2 and/or pH 6.0 and/or pH 5.8).

### Friability Measurement

This test for the robustness of the tablet is a standard friability test, namely the rotation of 20 tablets for 4 minutes at 25rpm in a friabulator (TAR 20 manufactured by ERWEKA). The number of capped or broken tablets was measured. The illustrative Examples indicate with a (√) that none of the tablets were capped or broken.

### Crushing Strength (N)

The crushing strength is a measure of the hardness of a tablet. It was measured by recording the diametrical crushing strength when the tablet was broken between the motorised jaws of a Schleuniger crushing strength tester. The range of crushing strengths of five tablets prepared with each Example formulation is given.

### Disintegration Time (Minutes)

The disintegration time was measured using the disintegration method described in the European Pharmacopoeia 1986, Ref V.5.1.1 (updated 1995) using tap water (pH approximately 7) as the liquid. The method measures the time (seconds) by which six tablets prepared with each Example formulation disintegrates.

### Examples 1-3

| **Excipient Standard Name** | **Example 1 (% w/w)** | **Example 2 (% w/w)** | **Example 3 (% w/w)** |
|---|---|---|---|
| Granular composition: | | | |
| Ibuprofen | 51.8 | 41.3 | 21.8 |
| Croscarmellose sodium | 5.2 | 4.1 | 3.3 |
| Tartaric acid | 20.8 | 16.5 | --- |
| Malic acid | --- | --- | 35 |
| Poloxamer | --- | --- | 0.2 |

| Extra-granular composition: | | | |
|---|---|---|---|
| Sodium bicarbonate | 21.6 | 37.7 | 39.5 |
| Colloidal silica | 0.3 | 0.2 | 0.1 |
| Sodium lauryl sulphate | 0.3 | 0.2 | 0.1 |

### Examples 1-3 (a): Preparation of Granular Component

In the process, the ibuprofen was melted by heating to approximately 75°C in a stainless steel vessel until fully molten. The acid component was then added to the molten ibuprofen and mixed for 5-10 minutes until uniformly dispersed. Other intragranular excipients may also be added at this stage. The molten mixture was then poured into a stainless steel tray and cooled over a period of up to 60 minutes, ensuring that a suspension was maintained. The mass formed was milled by passing through a cone mill having a screen with a round hole size of 1 mm, and the resulting granules were collected.

### Examples 1-3(b): Preparation of Tablets

The ingredients of the extra-granular composition are blended simultaneously with the intragranular composition for approximately 15 minutes in a blender. The blended material is fed to a tabletting machine and compressed into tablets containing 200mg ibuprofen.

### Results: Examples 1 - 3

| **Tablet Hardness and Disintegration Time** | | | |
|---|---|---|---|
| | **Example 1** | **Example 2** | **Example 3** |
| Tablet Hardness (Kp) | 4 | 5 | 4 |
| Disintegration time (seconds) | 180 | 180 | 60 |

### Examples 4-8

| **Excipient Standard Name** | **Example 4 (% w/w)** | **Example 5 (% w/w)** | **Example 6 (% w/w)** | **Example 7 (% w/w)** | **Example 8 (% w/w)** |
|---|---|---|---|---|---|
| Granular composition: | | | | | |
| Ibuprofen | 20 | 22 | 35 | 22 | 22 |
| Malic acid | 31 | --- | --- | --- | 35 |
| Citric acid | --- | 35 | --- | --- | --- |
| Tartaric acid | --- | --- | 28 | 35 | --- |
| Sodium stearyl fumarate | 0.5 | --- | --- | --- | --- |
| Poloxamer | --- | 0.2 | --- | --- | --- |

| Extra-granular composition: | | | | | |
|---|---|---|---|---|---|
| Sodium bicarbonate | 34.9 | 39.6 | 31.6 | 39.6 | 39.2 |
| Sodium starch glycolate | 3 | --- | --- | 3 | 3 |
| Croscarmellose sodium | --- | 3 | 5 | --- | --- |
| Sodium lauryl sulphate | 0.1 | 0.1 | 0.1 | 0.1 | 0.1 |
| Hydroxypropylme thyl cellulose | 0.1 | --- | --- | --- | --- |
| Sorbitol | 10 | --- | --- | --- | --- |
| Orange flavour | 0.4 | --- | --- | --- | 0.4 |
| Colloidal silica | --- | 0.1 | 0.1 | 0.1 | 0.1 |
| Poloxamer | --- | --- | 0.2 | 0.2 | 0.2 |

Examples 4-8 are prepared in the same manner as examples1-3. Tablets containing 200mg ibuprofen are prepared.

### Results: Examples 4-8

| **Dissolution Results at pH 7.2** | | | | | |
|---|---|---|---|---|---|
| **Time (min)** | **Ex 4** | **Ex 5** | **Ex 6** | **Ex 7** | **Ex 8** |
| 0 | 0 | 0 | 0 | 0 | 0 |
| 10 | 39.8 | 97.5 | 37.5 | 45.1 | 36.4 |
| 20 | 46.4 | 120.6 | 69.8 | 61.3 | 52.2 |
| 30 | 60.0 | 128.6 | 88.9 | 69.9 | 61.6 |
| 45 | 74.3 | 133.2 | 105.1 | 77.9 | 71.2 |
| 60 | 85.3 | 134.6 | 112.6 | 83.2 | 78.9 |

| **Tablet Hardness, Friability and Disintegration Time** | | | | | |
|---|---|---|---|---|---|
| | **Ex 4** | **Ex 5** | **Ex 6** | **Ex 7** | **Ex 8** |
| Tablet Hardness (Kp) | 11.1 | 23.6 | 10.8 | 6.3 | 11.1 |
| Friability (as a fraction of tablet number) | 0/10 | 0/10 | 0/10 | 8/10 | 1/10 |
| Disintegration time (seconds) | 900 | 600 | 1200 | 900 | 180 |

### Examples 9-11

| Excipient Standard Name | **Example 9 (% w/w)** | **Example 10 (% w/w)** | **Example 11 (% w/w)** |
|---|---|---|---|
| Granular composition: | | | |
| Ibuprofen | 22 | 22 | 20 |
| Malic acid | 35 | 35 | 31 |
| Sodium stearyl fumarate | --- | --- | 0.5 |
| Sodium starch glycolate | --- | 3 | --- |

| Extra-granular composition: | | | |
|---|---|---|---|
| Sodium bicarbonate | 38.4 | 38.5 | 34.9 |
| Sodium starch glycolate | 3 | --- | 3 |
| Microcrystalline cellulose | 1 | --- | --- |
| Hydroxypropylmethyl cellulose | --- | 0.9 | --- |
| Mannitol | --- | --- | 10 |
| Sodium lauryl sulphate | 0.1 | 0.1 | 0.1 |
| Colloidal silica | 0.1 | 0.1 | 0.1 |
| Orange flavour | 0.4 | 0.4 | 0.4 |

Examples 9-11 are prepared in the same manner as examples1-3. Tablets containing 200mg ibuprofen are prepared.

### Results: Examples 9-11

| **Dissolution Results at pH 7.2** | | | |
|---|---|---|---|
| **Time (min)** | **Ex 9** | **Ex 10** | **Ex 11** |
| 0 | 0 | 0 | 0 |
| 10 | 45.3 | 83.2 | 74.6 |
| 20 | 62.6 | 104.4 | 92.4 |
| 30 | 73.1 | 109.6 | 99.4 |
| 45 | 83.4 | 111.7 | 103.6 |
| 60 | 86.9 | 112.2 | 105.8 |

| **Tablet Hardness, Friability and Disintegration Time** | | | |
|---|---|---|---|
| | **Ex 9** | **Ex 10** | **Ex 11** |
| Tablet Hardness (Kp) | 11.8 | 11.0 | 8.9 |
| Friability (as a fraction of tablet number) | 0/10 | 0/10 | 0/10 |
| Disintegration time (seconds) | 600 | 360 | 210 |

### Examples 12 and 13

| **Excipient Standard Name** | **Example 12 (% w/w)** | **Example 13 (% w/w)** |
|---|---|---|
| Granular composition: | | |
| Flurbiprofen | 11 | --- |
| Naproxen | --- | 17 |
| Malic acid | 17 | 6 |

| Extra-granular excipients: | | |
|---|---|---|
| Sodium bicarbonate | 22 | 76.9 |
| Sodium starch glycolate | 50 | --- |
| Lactose | --- | 0.1 |

Examples 12 and 13 were prepared in the same manner as examples 1-3, except they contain the alternative NSAID's flurbiprofen or naproxen, instead of ibuprofen. In example 12 tablets containing 50mg flurbiprofen were prepared. In example 13 tablets containing 250mg naproxen were prepared.

### Results: Examples 12 and 13

| **Dissolution Results at pH 7.2** | | |
|---|---|---|
| **Time (min)** | **Ex 12** | **Ex 13** |
| 0 | 0 | 0 |
| 10 | 68.2 | 66.5 |
| 20 | 75.2 | 75.7 |
| 30 | 79.5 | 79.9 |
| 45 | 82.9 | 84.8 |
| 60 | 84.3 | 89.3 |

| **Tablet Hardness, Friability and Disintegration Time** | | |
|---|---|---|
| | **Ex 12** | **Ex 13** |
| Tablet Hardness (Kp) | 2.6 | 8.2 |
| Friability (as a fraction of tablet number) | 0/10 | 0/10 |
| Disintegration time (seconds) | 60 | 105 |

### Example 14

| **Excipient Standard Name** | **Example 14** |
|---|---|
| Granular composition: | |
| Ibuprofen | 19 |
| Malic acid | 30 |
| Croscarmellose sodium | 3 |
| Microcrystalline cellulose | 6.5 |

| Extra-granular excipients: | |
|---|---|
| Sodium bicarbonate | 34.8 |
| Sodium lauryl sulphate | 0.1 |
| Microcrystalline cellulose | 6.5 |
| Colloidal silica | 0.1 |

Example 14 was prepared in the same manner as examples 1- 3, using ibuprofen as the NSAID. Tablets containing 200mg ibuprofen were prepared.

### Results: Example 14

| **Dissolution Results at pH 7.2** | |
|---|---|
| **Time (min)** | **Example 14** |
| 0 | 0.0 |
| 10 | 81.0 |
| 20 | 103.1 |
| 30 | 115.1 |
| 45 | 125.2 |
| 60 | 131.0 |

| **Tablet Hardness, Friability and Disintegration Time** | |
|---|---|
| | **Example 14** |
| Tablet Hardness (Kp) | 14.9 |
| Friability (as a fraction of tablet number) | 0/10 |
| Disintegration time (seconds) | 105 |

### Examples 15 and 16

| **Excipient Standard Name** | **Example 15 (% w/w)** | **Example 16 (% w/w)** |
|---|---|---|
| Granular composition: | | |
| Ibuprofen | 22 | 35 |
| Citric acid | 35 | - |
| Tartaric acid | --- | 28 |
| Poloxamer | 0.2 | --- |

| Extra-granular composition: | | |
|---|---|---|
| Sodium bicarbonate | 39.6 | 33.6 |
| Croscarmellose sodium | 3 | 3 |
| Sodium lauryl sulphate | 0.1 | 0.1 |
| Colloidal silica | 0.1 | 0.1 |
| Poloxamer | --- | 0.2 |

### Example 15-16 (a): Preparation of Granule Format

In the practical process, the ibuprofen was melted by heating to approximately 75°C in a stainless steel vessel until fully molten. The acid component was then added to the molten ibuprofen and mixed for 5-10 minutes until uniformly dispersed. Other intragranular excipients may also be added at this stage. The molten mixture was then poured into a stainless steel tray and cooled over a period of up to 60 minutes, ensuring that a suspension was maintained. The mass formed was milled by passing through a cone mill having a screen with a round hole size of 1 mm, and the resulting granules were collected.

### Examples 15-16 (b)

The ingredients of the extra-granular composition were blended simultaneously with the intragranular composition for approximately 15 minutes in a blender. Granules containing 200mg ibuprofen are dispensed.

### Results: Examples 15-16

| **Dissolution Results at pH 7.2** | | |
|---|---|---|
| **Time (min)** | **Ex 15** | **Ex 16** |
| 0 | 0.0 | 0.0 |
| 10 | 76.2 | 79.1 |
| 20 | 93.5 | 96.7 |
| 30 | 95.0 | 103.4 |
| 45 | 102.2 | 108.2 |
| 60 | 101.8 | 109.3 |

### Examples 17-20

| **Excipient standard name** | **Example 17 (%w/w)** | **Example 18 (%w/w)** | **Example 19 (%w/w)** | **Example 20 (%w/w)** |
|---|---|---|---|---|
| Granular composition: | | | | |
| Ibuprofen | 21.5 | 21.5 | 19.6 | 19.6 |
| Malic acid | 34.7 | 34.7 | 31.3 | 31.3 |
| Sodium starch glycolate | 3.2 | 3.2 | 2.9 | 2.9 |
| Mannitol | --- | --- | 9.8 | --- |
| Microcrystalline cellulose | 1.0 | --- | --- | --- |
| Sorbitol | --- | --- | --- | 9.8 |
| Hydroxypropyl methyl cellulose | --- | 1.0 | --- | --- |

| Extra-granular composition: | | | | |
|---|---|---|---|---|
| Sodium bicarbonate | 39.0 | 39.0 | 35.3 | 35.3 |
| Sodium stearyl fumarate | --- | --- | 0.5 | 0.5 |
| Sodium lauryl sulphate | 0.1 | 0.1 | 0.1 | 0.1 |
| Hydroxypropyl methyl cellulose | --- | --- | --- | 0.1 |
| Colloidal silica | 0.1 | 0.1 | 0.1 | --- |
| Orange flavour | 0.4 | 0.4 | 0.4 | 0.4 |

### Example 17-20: Preparation of granule format

Examples 17-20 are prepared in the same manner as described in examples 1-3 except that the disintegrant (sodium starch glycolate) is used. Sweetners (mannitol and sorbitol) are used in examples 19 and 20 respectively. Diluent (microcrystalline cellulose) is used in example 17 and emulsifer (hydroxypropyl methylcellulose) is used in example 18.

### Examples 21-22

| **Excipient standard name** | **Example 21 (%w/w)** | **Example 22 (%w/w)** |
|---|---|---|
| Granular composition: | | |
| Ketoprofen | 5 | 10.2 |
| Malic acid | 80 | 32.8 |
| Mannitol | --- | 12.3 |

| Extra-granular composition: | | |
|---|---|---|
| Sodium bicarbonate | 15 | 41.0 |
| Croscarmellose sodium | --- | 2.1 |
| Sodium lauryl sulphate | --- | 0.4 |
| Orange flavour | --- | 1.2 |

### Example 21-22 (a): Preparation of granular component

Example 21-22 is prepared in the same manner as described in examples 1-3 except that the alternative NSAID ketoprofen is used, disintegrant (croscarmellose sodium) in example 22 is included in the extra-granular composition, no solubilizing agent is present and sweetner (mannitol) is used in example 22. The blended material is further processed:

### Example 21-22 (b): Preparation of tablets

Following the blending of the extra-granular and granular material, the blended material is fed to a tabletting machine and compressed into tablets containing 50mg ketoprofen.

### Examples 23-44

### Effervescent Granules

Each of the blended mixtures of the granular and extra-granular compositions prepared in each of Examples 1 to 22 may be filled into each sachets to provide a effervescent composition for addition to water to provide a liquid drink.

### Example 45

Each of the example compositions of Example 1 to 32 may be formulated to provide doses containing 50 mg, 100 mg, 300 mg and 400 mg ibuprofen (racemic and S(+)-ibuprofen)

### Examples 46-68

Each of the Examples 1 to 22 may be repeated, but with the basic component of the effervescent couple being included in the granular composition and the acid component of the effervescent couple being included in the extra-granular composition.

## Claims

1. An effervescent formulation comprising a non-steroidal anti-inflammatory drug (NSAID) and an effervescent couple comprising:
(a) a granular composition comprising a plurality of solidified melt granules of the NSAID with either an acidic or a basic component of the effervescent couple uniformly dispersed therein; and
(b) an extra-granular composition comprising the other of the basic or acidic component of the effervescent couple
wherein the NSAID is selected from racemic flurbiprofen, ibuprofen, ketoprofen, naproxen and salts thereof.

2. A formulation according to claim 1, wherein an acidic component of the effervescent couple is contained within the granular composition and a basic component of the effervescent couple is contained within the extra-granular composition.

3. A formulation according to claim 1, wherein a basic component of the effervescent couple is contained within the granular composition and an acidic component of the effervescent couple is contained within the extra-granular composition.

4. A formulation according to any one of the preceding claims, wherein the ratio of the effervescent couple to said non-steroidal anti-inflammatory drug is in the range 10: 1 to 1: 10 parts by weight.

5. A formulation according to any one of the preceding claims, comprising said effervescent couple in an amount of from 20 to 90% w/w.

6. A formulation according to any one of the preceding claims, comprising said acidic component in an amount of from 3 to 80% w/w,

7. A formulation according to any one of the preceding claims, comprising said basic component in an amount of from 9 to 90% w/w.

8. A formulation according to any of the preceding claims comprising racemic ibuprofen.

9. A formulation according to any one of the preceding claims, wherein the acidic component comprises at least one of citric acid, malic acid, tartaric acid, fumaric acid, ascorbic acid and adipic acid.

10. A formulation according to any one of the preceding claims, wherein the basic component comprises at least one of sodium carbonate, sodium bicarbonate, potassium carbonate and potassium bicarbonate.

11. A formulation according to any one of the preceding claims, further comprising a disintegrant.

12. A formulation according to claim 11, wherein the granular composition comprises from 1 to 50% w/w disintegrant.

13. A formulation according to claim 11 or 12, wherein the disintegrant comprises at least one of croscarmellose sodium and sodium starch glycolate.

14. A formulation according to any one of the preceding claims, wherein the extra-granular composition comprises silicon dioxide.

15. A formulation according to any one of the preceding claims, further comprising a surfactant.

16. A formulation according to any one of the preceding claims further comprising a diluent in an amount of from 0.1 to 60% w/w.

17. A formulation according to any one of the preceding claims, wherein the granular composition comprises:
(a) 5-40% w/w non-steroidal anti-inflammatory drug;
(b) 20-80% w/w acidic component; and
(c) 0.1-15% w/w disintegrant.

18. A formulation according to any one of the preceding claims comprising:
(a) 10-25% w/w ibuprofen;
(b) 25-40% w/w acidic component;
(c) 0.5-10% w/w croscarmellose sodium;
(d) 25-40% w/w sodium bicarbonate; and
(e) 0.05%-5% w/w surfactant.

19. A granular composition comprising a plurality of solidified melt granules of a non-steroidal anti-inflammatory drug and an edible organic acid.

20. A granular composition according to claim 19, further comprising a disintegrant.

21. A process for the preparation of a formulation according to any one of claims 1 to 18, comprising:
(a)
(i) combining an acidic or basic component of an effervescent couple with the NSAID in the solid state and heating the mixture until the NSAID is molten; or
(ii) heating the NSAID until it is molten and combining an acidic or basic component of an effervescent couple with the molten NSAID. to form a mixture thereof:
(b) forming the mixture into solidified melt granules;
(c) combining said solidified melt granules with an extra-granular composition comprising the other of the basic or acidic component of the effervescent couple.

22. A process according to claim 21, in which an acidic component and the NSAID are uniformly mixed in the solid state prior to heating.

23. A process according to claim 21, comprising:
a) heating the NSAID until it is molten and then combining the molten NSAID with an acidic component of an effervescent couple to form a mixture thereof;
b) forming said mixture into solidified melt granules with cooling;
c) combining said solidified melt granules with an extra-granular composition comprising a basic component of an effervescent couple.

24. A process according to any one of claims 21 to 23, wherein the mixture is cooled to the solid state before being formed into granules,

25. A process according to any one of claims 21 to 24, wherein the extra-granular component further comprises at least one of silicon dioxide, stearic acid or a salt thereof, a surfactant and a diluent.

26. A process for the preparation of a granular composition according to claim 19 or 20, comprising:
(a)
(i) combining the acidic component of an effervescent couple with the NSAID in the solid state and heating the mixture until the NSAID is molten; or
(ii) heating the NSAID until it is molten and combining the acidic component of an effervescent couple with the molten NSAID to form a mixture thereof;
(b) forming a uniform mixture; and
(c) forming said mixture into solidified melt granules with cooling.

27. A process according to claim 26, wherein the acidic component and the NSAID are uniformly mixed in the solid state prior to heating.

28. A process according to claim 26, comprising:
(a) heating the NSAID until it is molten and then combining the molten NSAID with an acidic component of an effervescent couple to form a mixture thereof;
(b) forming a uniform mixture; and
(c) forming said mixture into solidified melt granules with cooling.

29. A process according to any one of claims 26 to 28, wherein the uniform mixture of step (b) is cooled to the solid state before being formed into granules.

30. A process according to any one of claims 21 to 29, wherein the molten NSAID is obtained by melt-extrusion.

31. A process according to any one of claims 21 to 30, wherein a temperature of from 80 to 130° C is used in the heating step.

32. A process according to any one of claims 21 to 31, wherein the NSAID is melted completely during the heating step.

33. A process according to any one of claims 21 to 32, wherein the molten mixture solidifies ir 5 minutes or less.

34. A process according to any one claims 21 to 33, wherein a disintegrant is combined with the NSAID in step (a).

35. A process according to any one of claims 21 to 34, wherein at least one of a surfactant and a diluent is combined with the NSAID in step (a).

36. A process according to any one of claims 21 to 35, wherein the NSAID comprises ibuprofen,

37. A formulation according to any one of claims 1 to 18 in the form of a unit dose.

38. A formulation according to any one of claims 1 to 18 and 37 in the form of a powder mixture or a tablet.

39. A formulation according to any one claims 1 to 18, 37 and 38 comprising:
(a) 10-90% w/w melt granules; and
(b) 90-10% w/w extra-granular composition.

40. A formulation according to claim 39 comprising:
(a) 40-80% w/w melt granules consisting essentially of said NSAID, said acidic component ard a disintegrant;
(b) 20-60% w/w extra-granular composition comprising a base in an amount greater than 90% of said extra-granular composition.

41. The use of a formulation according to any one of claims 1 to 18 or a granular composition according to claim 19 or 20 in the manufacture of a medicament for the treatment of pain and/or inflammation and/or fever.

42. Use according to claim 41 in the manufacture of a medicament for the treatment of coughs, colds, influenza, migraine, headache, rheumatic pain, muscular pain and neuralgia.

43. A formulation according to any one of claims 1 to 18 or a granular composition according to claim 19 or 20 for use in the treatment of pain and/or inflammation and/or fever.

## Patentansprüche

1. Brauseformulierung, umfassend einen nichtsteroidalen entzündungshemmenden Wirkstoff (Non-Steroidal Anti-Inflammatory Drug, NSAID) und ein Brausepaar, umfassend:
(a) eine granulären Zusammensetzung, umfassend mehrere verfestigte Schmelzgranulate des NSAID mit, einheitlich dispergiert darin, entweder einer sauren oder einer basischen Komponente des Brausepaars, und
(b) eine extragranuläre Zusammensetzung, umfassend die andere der basischen bzw. sauren Komponente des Brausepaars,
wobei der NSAID ausgewählt ist aus racemischem Flurbiprofen, Ibuprofen, Ketoprofen, Naproxen und Salzen davon.

2. Formulierung nach Anspruch 1, wobei eine saure Komponente des Brausepaars in der granulären Zusammensetzung enthalten ist und eine basische Komponente des Brausepaars in der extragranulären Zusammensetzung enthalten ist.

3. Formulierung nach Anspruch 1, wobei eine basische Komponente des Brausepaars in der granulären Zusammensetzung enthalten ist und eine saure Komponente des Brausepaars in der extragranulären Zusammensetzung enthalten ist.

4. Formulierung nach einem der vorhergehenden Ansprüche, wobei das Verhältnis von Brausepaar zum nichtsteroidalem entzündungshemmenden Wirkstoff im Bereich von 10:1 bis 1:10 Gewichtsteilen liegt.

5. Formulierung nach einem der vorhergehenden Ansprüche, welche das Brausepaar in einer Menge von 20 bis 90 Gew.-% umfasst.

6. Formulierung nach einem der vorhergehenden Ansprüche, welche die saure Komponente in einer Menge von 3 bis 80 Gew.-% umfasst.

7. Formulierung nach einem der vorhergehenden Ansprüche, welche die basische Komponente in einer Menge von 9 bis 90 Gew.-% umfasst.

8. Formulierung nach einem der vorhergehenden Ansprüche, welche racemisches Ibuprofen umfasst.

9. Formulierung nach einem der vorhergehenden Ansprüche, wobei die saure Komponente mindestens eine der Folgenden umfasst: Citronensäure, Äpfelsäure, Weinsäure, Fumarsäure, Ascorbinsäure und Adipinsäure.

10. Formulierung nach einem der vorhergehenden Ansprüche, wobei die basische Komponente mindestens eine der folgenden Verbindungen umfasst: Natriumcarbonat, Natriumhydrogencarbonat, Kaliumcarbonat und Kaliumhydrogencarbonat.

11. Formulierung nach einem der vorhergehenden Ansprüche, welcher weiterhin ein Sprengmittel umfasst.

12. Formulierung nach Anspruch 11, wobei die granuläre Zusammensetzung 1 bis 50 Gew.-% Sprengmittel umfasst.

13. Formulierung nach Anspruch 11 oder 12, wobei das Sprengmittel mindestens eine der folgenden Verbindungen umfasst: Croscarmellose-Natrium und Natriumstärkeglykolat.

14. Formulierung nach einem der vorhergehenden Ansprüche, wobei die extragranuläre Zusammensetzung Siliciumdioxid umfasst.

15. Formulierung nach einem der vorhergehenden Ansprüche, welche weiterhin ein Tensid umfasst.

16. Formulierung nach einem der vorhergehenden Ansprüche, welche weiterhin ein Verdünnungsmittel in einer Menge von 0,1 bis 60 Gew.-% umfasst.

17. Formulierung nach einem der vorhergehenden Ansprüche, wobei die granuläre Zusammensetzung Folgendes umfasst:
(a) 5-40 Gew.-% nichtsteroidalen entzündungshemmenden Wirkstoff;
(b) 20-80 Gew.-% saure Komponente und
(c) 0,1-15 Gew.-% Sprengmittel.

18. Formulierung nach einem der vorhergehenden Ansprüche, welche Folgendes umfasst:
(a) 10-25 Gew.-% Ibuprofen;
(b) 25-40 Gew.-% saure Komponente;
(c) 0,5-10 Gew.-% Croscarmellose-Natrium;
(d) 25-40 Gew.-% Natriumhydrogencarbonat und
(e) 0,05-5 Gew.-% Tensid.

19. Granuläre Zusammensetzung, umfassend eine Mehrzahl verfestigter Schmelzgranulate eines nichtsteroidalen entzündungshemmenden Wirkstoffs und einer essbaren organischen Säure.

20. Granuläre Zusammensetzung nach Anspruch 19, welche weiterhin ein Sprengmittel umfasst.

21. Verfahren zur Herstellung einer Formulierung nach einem der Ansprüche 1 bis 18, welche Folgendes umfasst:
(a)
(i) Kombinieren einer sauren oder basischen Komponente eines Brausepaars mit dem NSAID in festem Zustand und Erhitzen der Mischung, bis der NSAID geschmolzen ist, oder
(ii) Erhitzen des NSAID, bis er geschmolzen ist, und Kombinieren einer sauren oder basischen Komponente eines Brausepaares mit dem geschmolzenen NSAID unter Bildung einer Mischung davon,
(b) Formen der Mischung zu einem verfestigten Schmelzgranulat;
(c) Kombinieren des verfestigten Schmelzgranulats mit einer extragranulären Zusammensetzung, welche die andere der basischen bzw. sauren Komponente des Brausepaars umfasst.

22. Verfahren nach Anspruch 21, bei dem man vor dem Erhitzen eine saure Komponente und den NSAID im festen Zustand einheitlich mischt.

23. Verfahren nach Anspruch 21, welches Folgendes umfasst:
a) Erhitzen des NSAID, bis er geschmolzen ist, und anschließendes Kombinieren des geschmolzenen NSAID mit einer sauren Komponente eines Brausepaares unter Bildung einer Mischung davon;
b) Formen der Mischung zu einem verfestigten Schmelzgranulat unter Abkühlen;
c) Kombinieren des verfestigten Schmelzgranulats mit einer extragranulären Zusammensetzung, welche eine basische Komponente eines Brausepaares umfasst.

24. Verfahren nach einem der Ansprüche 21 bis 23, wobei man die Mischung vor dem Formen zu einem Granulat auf den festen Zustand abkühlt.

25. Verfahren nach einem der Ansprüche 21 bis 24, wobei die extragranuläre Komponente weiterhin mindestens einen der folgenden Bestandteile umfasst: Siliciumdioxid, Stearinsäure oder ein Salz davon, ein Tensid und ein Verdünnungsmittel.

26. Verfahren zur Herstellung einer granulären Zusammensetzung nach Anspruch 19 oder 20, welches Folgendes umfasst:
(a)
(i) Kombinieren der sauren Komponente eines Brausepaares mit dem NSAID in festem Zustand und Erhitzen der Mischung, bis der NSAID geschmolzen ist, oder
(ii) Erhitzen des NSAID, bis er geschmolzen ist, und Kombinieren der sauren Komponente eines Brausepaares mit dem geschmolzenen NSAID unter Bildung einer Mischung davon;
(b) Bildung einer einheitlichen Mischung und
(c) Formen der Mischung zu einem verfestigten Schmelzgranulat unter Abkühlen.

27. Verfahren nach Anspruch 26, wobei man die saure Komponente und den NSAID vor dem Erhitzen einheitlich im festen Zustand mischt.

28. Verfahren nach Anspruch 26, welches Folgendes umfasst:
(a) Erhitzen des NSAID, bis er geschmolzen ist, und anschließendes Kombinieren des geschmolzenen NSAID mit einer sauren Komponente eines Brausepaars unter Bildung einer Mischung davon;
(b) Bildung einer einheitlichen Mischung und
(c) Formen der Mischung zu einem verfestigten Schmelzgranulat unter Abkühlen.

29. Verfahren nach einem der Ansprüche 26 bis 28, bei dem man die einheitlich Mischung von Schritt (b) vor dem Formen zu Granulat auf den festen Zustand abkühlt.

30. Verfahren nach einem der Ansprüche 21 bis 29, wobei man den geschmolzenen NSAID durch Schmelzextrusion erhält.

31. Verfahren nach einem der Ansprüche 21 bis 30, wobei die in dem Erhitzungsschritt verwendete Temperatur 80 bis 130°C beträgt.

32. Verfahren nach einem der Ansprüche 21 bis 31, wobei der NSAID während des Erhitzungsschritts vollständig geschmolzen wird.

33. Verfahren nach einem der Ansprüche 21 bis 32, wobei die geschmolzene Mischung sich innerhalb von 5 Minuten oder weniger verfestigt.

34. Verfahren nach einem der Ansprüche 21 bis 33, wobei man in Schritt (a) ein Sprengmittel mit dem NSAID kombiniert.

35. Verfahren nach einem der Ansprüche 21 bis 34, bei dem man in Schritt (a) mindestens eine der Komponenten Tensid und Verdünnungsmittel mit dem NSAID kombiniert.

36. Verfahren nach einem der Ansprüche 21 bis 35, wobei der NSAID Ibuprofen umfasst.

37. Formulierung nach einem der Ansprüche 1 bis 18 in Form einer Einheitsdosis.

38. Formulierung nach einem der Ansprüche 1 bis 18 und 37 in Form einer Pulvermischung oder einer Tablette.

39. Formulierung nach einem der Ansprüche 1 bis 18, 37 und 38, welche Folgendes umfasst:
(a) 10-90 Gew.-% Schmelzgranulat und
(b) 90-10 Gew.-% extragranuläre Zusammensetzung.

40. Formulierung nach Anspruch 39, welche Folgendes umfasst:
(a) 40-80 Gew.-% Schmelzgranulat, welches im Wesentlichen aus dem NSAID, der sauren Komponente und einem Sprengmittel besteht;
(b) 20-60 Gew.-% der extragranulären Zusammensetzung, welche eine Base in einer Menge von mehr als 90% der extragranulären Zusammensetzung umfasst.

41. Verwendung einer Formulierung nach einem der Ansprüche 1 bis 18 oder einer granulären Zusammensetzung nach Anspruch 19 oder 20 zur Herstellung eines Medikaments zur Behandlung von Schmerzen und/oder Entzündung und/oder Fieber.

42. Verwendung nach Anspruch 41 zur Herstellung eines Medikaments zur Behandlung von Husten, Erkältungen, Grippe, Migräne, Kopfschmerzen, rheumatischen Schmerzen, Muskelschmerzen und Neuralgie.

43. Formulierung nach einem der Ansprüche 1 bis 18 oder granuläre Zusammensetzung nach Anspruch 19 oder 20 zur Verwendung bei der Behandlung von Schmerzen und/oder Entzündung und/oder Fieber.

## Revendications

1. Formulation effervescente comprenant un médicament anti-inflammatoire non stéroïdien (NSAID) et un couple effervescent comprenant :
(a) une composition granulaire comprenant une pluralité de granules de matière fondue solidifiée du NSAID avec un composant acide ou basique du couple effervescent uniformément dispersé dans celle-ci ; et
(b) une composition extragranulaire comprenant l'autre du composant basique ou acide du couple effervescent dans laquelle le NSAID est choisi parmi le racémique de flurbiprofène, ibuprofène, kétoprofène, naproxène et des sels de ceux-ci.

2. Formulation selon la revendication 1, dans laquelle un composant acide du couple effervescent est contenu dans la composition granulaire et un composant basique du couple effervescent est contenu dans la composition extragranulaire.

3. Formulation selon la revendication 1, dans laquelle un composant basique du couple effervescent est contenu dans la composition granulaire et un composant acide du couple effervescent est contenu dans la composition extragranulaire.

4. Formulation selon l'une quelconque des revendications précédentes, dans laquelle le rapport du couple effervescent audit médicament anti-inflammatoire non stéroïdien est dans la plage de 10:1 à 1:10 parties en poids.

5. Formulation selon l'une quelconque des revendications précédentes, comprenant ledit couple effervescent en une quantité de 20 à 90 % m/m.

6. Formulation selon l'une quelconque des revendications précédentes, comprenant ledit composant acide en une quantité de 3 à 80 % m/m.

7. Formulation selon l'une quelconque des revendications précédentes, comprenant ledit composant basique en une quantité de 9 à 90 % m/m.

8. Formulation selon l'une quelconque des revendications précédentes comprenant le racémique d'ibuprofène.

9. Formulation selon l'une quelconque des revendications précédentes, dans laquelle le composant acide comprend au moins l'un de l'acide citrique, l'acide Malik, l'acide tartrique, l'acide fumarique, l'acide ascorbique et l'acide adipique.

10. Formulation selon l'une quelconque des revendications précédentes, dans laquelle le composant basique comprend au moins l'un des carbonates de sodium, bicarbonate de sodium, carbonate de potassium et bicarbonate de potassium.

11. Formulation selon l'une quelconque des revendications précédentes, comprenant en outre un délitant.

12. Formulation selon la revendication 11, dans laquelle la composition granulaire comprend de 1 à 50 % m/m de délitant.

13. Formulation selon la revendication 11 ou 12, dans laquelle le délitant comprend au moins l'un de la croscarmellose sodique et le glycolate d'amidon sodique.

14. Formulation selon l'une quelconque des revendications précédentes, dans laquelle la composition extragranulaire comprend du dioxyde de silicium.

15. Formulation selon l'une quelconque des revendications précédentes, comprenant en outre un tensioactif.

16. Formulation selon l'une quelconque des revendications précédentes comprenant en outre un diluant en une quantité de 0,1 à 60 % m/m.

17. Formulation selon l'une quelconque des revendications précédentes, dans laquelle la composition granulaire comprend :
(a) de 5 à 40 % m/m de médicament anti-inflammatoire non stéroïdien ;
(b) de 20 à 80 % m/m de composant acide ; et
(c) de 0,1 à 15 % m/m de délitant.

18. Formulation selon l'une quelconque des revendications précédentes comprenant :
(a) de 10 à 25 % m/m d'ibuprofène ;
(b) de 25 à 40 % m/m de composant acide ;
(c) de 0,5 à 10 % m/m de croscarmellose sodique ;
(d) de 25 à 40 % m/m de bicarbonate de sodium ; et
(e) de 0,05 % à 5 % m/m de tensioactif.

19. Composition granulaire comprenant une pluralité de granules de matière fondue solidifiée d'un médicament anti-inflammatoire non stéroïdien et un acide organique comestible.

20. Composition granulaire selon la revendication 19, comprenant en outre un délitant.

21. Procédé de préparation d'une formulation selon l'une quelconque des revendications 1 à 18 comprenant :
(a)
(i) la combinaison d'un composant acide ou basique d'un couple effervescent avec le NSAID à l'état solide et le chauffage du mélange jusqu'à ce que le NSAID soit fondu ; ou
(ii) le chauffage du NSAID jusqu'à ce qu'il soit fondu et la combinaison d'un composant acide ou basique d'un couple effervescent avec le NSAID pour former un mélange de ceux-ci ;
(b) la formation du mélange en granules de matière fondue solidifiée ;
(c) la combinaisons desdits granules de matière fondue solidifiée avec une composition extragranulaire comprenant l'autre du composant basique ou acide du couple effervescent.

22. Procédé selon la revendication 21, dans lequel un composant acide et le NSAID sont uniformément mélangés à l'état solide avant chauffage.

23. Procédé selon la revendication 21, comprenant :
a) le chauffage du NSAID jusqu'à ce qu'il soit fondu et ensuite la combinaison du NSAID fondu avec un composant acide d'un couple effervescent pour former un mélange de ceux-ci ;
b) la formation dudit mélange en granules de matière fondue solidifiée avec refroidissement ;
c) la combinaison desdits granules de matière fondue solidifiée avec une composition extragranulaire comprenant un composant basique d'un couple effervescent.

24. Procédé selon l'une quelconque des revendications 21 à 23, dans lequel le mélange est refroidi à l'état solide avant d'être formé en granules.

25. Procédé selon l'une quelconque des revendications 21 à 24, dans lequel le composant extragranulaire comprend en outre au moins l'un des dioxyde de silicium, acide stéarique ou un sel de celui-ci, un tensioactif et un diluant.

26. Procédé de préparation d'une composition granulaire selon la revendication 19 ou 20, comprenant :
(a)
(i) la combinaison du composant acide d'un couple effervescent avec le NSAID à l'état solide et le chauffage du mélange jusqu'à ce que le NSAID soit fondu ; ou
(ii) le chauffage du NSAID jusqu'à ce qu'il soit fondu et la combinaison du composant acide d'un couple effervescent avec le NSAID fondu pour former un mélange de ceux-ci ;
(b) la formation d'un mélange uniforme ; et
(c) la formation dudit mélange en granules de matière fondue solidifiée avec refroidissement.

27. Procédé selon la revendication 26, dans lequel le composant acide et le NSAID sont uniformément mélangés à l'état solide avant chauffage.

28. Procédé selon la revendication 26, comprenant :
(a) le chauffage du NSAID jusqu'à ce qu'il soit fondu et ensuite la combinaison du NSAID fondu avec un composant acide d'un couple effervescent pour former un mélange de ceux-ci ;
(b) la formation d'un mélange uniforme ; et
(c) la formation dudit mélange en granules de matière fondue solidifiée avec refroidissement.

29. Procédé selon l'une quelconque des revendications 26 à 28, dans lequel le mélange uniforme de l'étape (b) est refroidi à l'état solide avant d'être formé en granules.

30. Procédé selon l'une quelconque des revendications 21 à 29, dans lequel le NSAID fondu est obtenu par extrusion de matière fondue.

31. Procédé selon l'une quelconque des revendications 21 à 30, dans lequel une température de 80 à 130 °C est utilisée dans l'étape de chauffage.

32. Procédé selon l'une quelconque des revendications 21 à 31, dans lequel le NSAID est totalement fondu pendant l'étape de chauffage.

33. Procédé selon l'une quelconque des revendications 21 à 32, dans lequel le mélange fondu solidifie en 5 minutes au moins.

34. Procédé selon l'une quelconque des revendications 21 à 33, dans lequel un délitant est combiné avec le NSAID dans l'étape (a).

35. Procédé selon l'une quelconque des revendications 21 à 34, dans lequel au moins l'un d'un tensioactif et un diluant est combiné avec le NSAID dans l'étape (a).

36. Procédé selon l'une quelconque des revendications 21 à 35, dans lequel le NSAID comprend de l'ibuprofène.

37. Formulation selon l'une quelconque des revendications 1 à 18 sous la forme d'une dose unitaire.

38. Formulation selon l'une quelconque des revendications 1 à 18 et 37 sous la forme d'un mélange en poudre ou d'un comprimé.

39. Formulation selon l'une quelconque des revendications 1 à 18, 37 et 38 comprenant :
(a) de 10 à 90 % m/m de granules de matière fondue ; et
(b) de 90 à 10 % m/m de composition extragranulaire.

40. Formulation selon la revendication 39 comprenant :
(a) de 40 à 80 % m/m de granules de matière fondue essentiellement constitués dudit NSAID, dudit composant acide et d'un délitant ;
(b) de 20 à 60 % m/m de composition extragranulaire comprenant une base en une quantité supérieure à 90 % de ladite composition extragranulaire.

41. Utilisation d'une formulation selon l'une quelconque des revendications 1 à 18 ou d'une composition granulaire selon la revendication 19 ou 20 dans la fabrication d'un médicament pour le traitement de la douleur et/ou l'inflammation et/ou la fièvre.

42. Utilisation selon la revendication 41 dans la fabrication d'un médicament pour le traitement de la toux, d'urine, de la grippe, de la migraine, des céphalées, de la douleur rhumatismale, de la douleur musculaire et de la névralgie.

43. Formulation selon l'une quelconque des revendications 1 à 18 ou composition granulaire selon la revendication 19 ou 20 pour utilisation dans le traitement de la douleur et/ou l'inflammation et/ou la fièvre.
